# EUROPEAN PATENT SPECIFICATION

(11) **EP 1 330 999 B1**
(45) Date of publication and mention of the grant of the patent: **26.12.2007**
(21) Application number: 01974762.5
(22) Date of filing: 10.10.2001
(51) Int. Cl.: A61K 8/63, A61Q 19/02, A61K 31/58

(54) **MELANOGENESIS INHIBITORS AND WHITENING AGENTS COMPRISING ERGOSTEROL DERIVATIVE AND COMPOSITIONS CONTAINING ERGOSTEROL DERIVATIVE**
MELANOGENESE-HEMMER UND AUFHELLUNGSMITTEL MIT EINEM ERGOSTEROL-DERIVAT UND EIN ERGOSTEROL-DERIVAT ENTHALTENDE ZUSAMMENSETZUNGEN
INHIBITEURS DE LA MELANOGENESE ET AGENT BLANCHISSANT RENFERMANT UN DERIVE D'ERGOSTEROL ET COMPOSITIONS RENFERMANT CE DERIVE

(30) Priority: 11.10.2000 JP 2000310290
(43) Date of publication of application: 30.07.2003
(73) Proprietor: Sakamoto Bio Co. Ltd., Akita -shi 010-1233 (JP)
(72) Inventor: SAKAMOTO, Kenji, c/o, Sakamoto Bio Co., Ltd., Kawabe-gun, Akita 010-1233 (JP); HATA, Keishi, Akita-shi, Akita 010-0973 (JP)
(74) Representative: Hallybone, Huw George
(86) International application number: PCT/JP2001/008892
(87) International publication number: WO 2002/030366

(56) References cited:
- WO-A-99/21961
- JP-A- 2 049 710
- JP-A- 7 316 035
- JP-A- 58 113 118
- JP-A- 2000 136 130
- JP-A- 2000 319 192
- DATABASE EMBASE [Online] ELSEVIER SCIENCE PUBLISHERS, AMSTERDAM, NL; 2001, GAO J-M ET AL: "A new ceramide from the basidiomycete Russula cyanoxantha" XP002317092 Database accession no. EMB-2001102408 & LIPIDS 2001 UNITED STATES, vol. 36, no. 2, 2001, pages 175-180, ISSN: 0024-4201
- PATENT ABSTRACTS OF JAPAN vol. 014, no. 342 (C-0743), 24 July 1990 (1990-07-24) & JP 02 124809 A (ERUSORU PROD KK), 14 May 1990 (1990-05-14)
- PATENT ABSTRACTS OF JAPAN vol. 016, no. 156 (C-0930), 16 April 1992 (1992-04-16) & JP 04 009321 A (SUNSTAR INC), 14 January 1992 (1992-01-14)
- PATENT ABSTRACTS OF JAPAN vol. 018, no. 131 (C-1175), 3 March 1994 (1994-03-03) & JP 05 317016 A (TANABE SEIYAKU CO LTD; others: 01), 3 December 1993 (1993-12-03)
- PHYTOCHEMISTRY, vol. 49, no. 6, 1998, pages 1651-1657, XP002317091
- ISHIZUKA TAKAAKI ET AL.: 'Sterol constituents from the fruit bodies of grifola frondosa(FR.) S.F. GRAY' CHEM. PHARM. BULL. vol. 45, no. 11, 1997, pages 1756 - 1760, XP002907828
- YASUKAWA KEN ET AL.: 'Inhibitory effects of ergosterol isolated from the edible mushroom hypsizigus marmoreus on TPA-induced inflammatory ear oedema and tumour promotion in mice' PHYTOTHERAPY RESEARCH vol. 8, no. 1, 1994, pages 10 - 13, XP002907829

## Description

### FIELD OF THE INVENTION

The present invention relates to melanogenesis inhibitors and whitening agents comprising an ergosterol derivative particularly to applications thereof in cosmetics, drugs, dermatological preparations, and foods. The invention further relates to melanogenesis inhibitors and to whitening agents, particularly to cosmetics, drugs, dermatological preparations, and foods which are capable of inhibiting melanogenesis in the melanocytes, whitening the sun-burnt skin after exposure to ultraviolet rays and preventing, ameliorating, and treating skin pigmentation such as spots, freckles, liver spots, and the like caused by sunburn or the like.

### BACKGROUND OF THE INVENTION

Skin pigmentation such as spots, freckles, and liver spots caused by sunburn and the like increases or becomes harder to fade with aging, a problem which annoys the middle-aged and older. Such pigmentation, for which the mechanism is yet to be established, is believed to be caused by inflammation of the skin as induced by sun light (in particular, ultraviolet rays) or the like, whereby melanin pigment is produced by the melanin producing granule, which is called melanosome, in the melanocytes present in the epidermal cells and is delivered to adjacent cells.

The above problem has prompted development of a substance capable of modifying such pigmentation and restoring a normal skin color , resulting in many commercial products JP-A-2000-319192, JP-A-7-316035, JP-A-2-49710 and JP-A-58-113118 describe compositions comprising an extract of Gandoderma Lucidum and Grifola Fodonsa to shown an effect of prohibiting melaninformation and to show a skin whitening effect. Other well-known example of commercial products include L-ascorbic acid, kojic acid, hydroquinone, and the like.

However, L-ascorbic acid is deficient in stability, and kojic acid is known to be effective, but weak in depigmentation activity. While hydroquinone also exhibits good depigmentation activity, it is problematic for use in pharmaceutical preparation because of its tendencies to irritate the skin and cause allergic reactions. Accordingly, at present there is no known agent highly effective for preventing pigmentation and for ameliorating the problem.

### SUMMARY OF THE INVENTION

It is an object of the present invention, which addresses the above-mentioned problem, to provide a substance which is efficacious in whitening sun-burnt skin after exposure to ultraviolet rays and preventing, ameliorating, and treating skin pigmentation such as spots, freckles, and liver spots caused by sunburn or the like.

The present inventors studied a way to solve the above problem and discovered that an extract of the antler-shaped [fruiting body] of the *Ganoderma lucidum* is effective for inhibiting melanogenesis, and that an ergosterol derivative having a specific structure isolated from said extract by means of separation and purification is highly effective for inhibiting melanogenesis and skin whitening thereby overcoming the above problem, which has led to the present invention.

The present invention relates to the use of a melanogenesis inhibitor and whitening agent which are comprised of an ergosterol derivative represented by Formula (1) below:

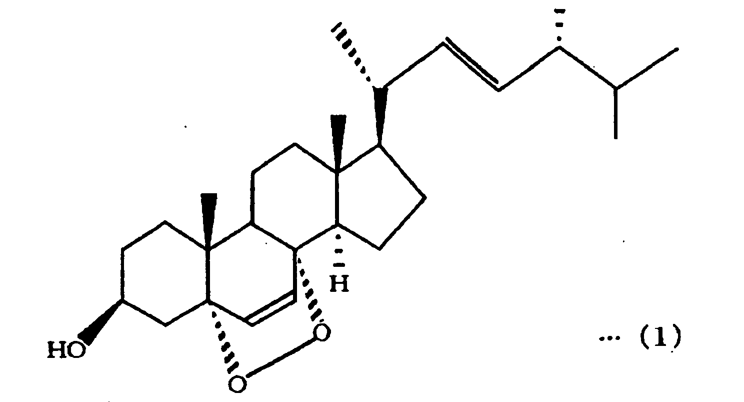

### DETAILED DESCRIPTION OF THE PREFERRED EMBODIMENT

The present invention will be described in detail hereinafter. The ergosterol derivative represented by Formula (1) used in this invention, 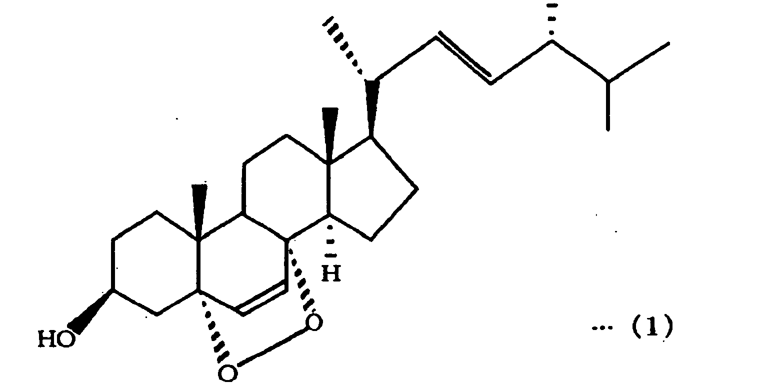 a known substance, which has heretofore been known to be isolable from a mushroom commonly referred to as "maitake" (Takaaki Ishizuka, et al Chem. Pharm. Bull. 45(11)1756-1760(1997)). However, it has not been known that the ergosterol derivative represented by Formula (1) (hereafter, referred to as Formula (1)) is effective for inhibiting melanogenesis and for skin whitening.

Formula (1), which can be isolated from fungi, is made available from any fungus which contains Formula (1), preferably from the antler shaped fruiting body of the *Ganoderma lucidum*. Alternatively, Formula (1) may be chemically synthesized. Formula (1) may be isolated from any fungus containing Formula (1) as explained below. Portions from the fruiting body or mycelium, spore, or the like of fungus are preferably chopped, immersed in an extracting solvent or heated to reflux therein, and filtered or centrifuged off insoluble material, resulting in an extract solution, which is optionally concentrated and subjected to known means of separation and purification for isolation.

Solvents commonly used for extraction may also be utilized which include organic solvents such as alcohols such as methanol, ethanol, and the like, acetone, ethyl acetate, and the like as well as water either alone or in combination. A conventional extraction procedure may be used, generally calling for an extraction at a temperature ranging from 0 to 100°C, preferably from 40 to 70°C for a duration ranging from 1 to 168 hrs, preferably24 to 72 hours. The extract solution is subjected to an isolation process, which may include chromatographic processes such as reverse phase chromatography, gel filtration column chromatography, liquid phase chromatography, and the like.

Specific examples for extraction from fungi and isolation of Formula (1) are described below:

### Example for isolation

Specimens of the distinctively antler-shaped fruiting body of the *Ganoderma lucidum* collected from its artificially cultured variety were chopped and immersed for extraction in ethanol for 72 hours at 40 °C. After removal of the insoluble matter by filtration and concentration of the resultant extract solution, the crude product was purified using reverse phase chromatography. The structure of the isolated substance was determined using GC/MS(mass spectroscopy) and NMR. The substance was identified as 5α,8α-epidioxy-(22E,24R)-orgosta-6,22-dien-3β-ol as represented by Formula (1).

Formula (1) of this invention, as will be described later, is effective for inhibiting melanogenesis and for skin whitening. Thus, the composition containing Formula (1) as specified in this invention is applicable to cosmetics, pharmaceutical products, and foods which function to prevent and treat symptoms related to melanogenesis inhibition and to skin whitening.

The composition used in the method of the present invention may be administered either orally (internally) or topically(externally). The composition to be orally administered calls for a preparation, for example, in the form of pharmaceutical products or foods. The composition to be topically administered calls for a preparation in the form of cosmetics, medicated cosmetics, drugs, dermatological preparations, or the like.

Although it will be most preferred, as a general rule, to use an isolated form of Formula (1) for formulating the composition of this invention, it may also be formulated in the form of an extract from Formula (1)-containing plants, fungi, and the like.

### Pharmaceuticals,

The compositions used in the present invention containing Formula (1) are further detailed according to their use.

Cosmetics, the first application of the present invention, are broadly applicable as those including medicated cosmetics, such as dermatological preparations; they are , for example, ointments, solutions, creams, emulsions, toners, lotions, gels, essences, foundations, pack-masks, lipsticks, sticks, bath preparations, and the like.

The cosmetic form can encompass a broad range of formulation types such as solution, solubilized formula, powder, powder dispersion, oily solution, gel, ointment, aerosol, water-in-oil, water-in-oil-in-solid types, and the like.

Formula (1) may be present in the compositions used in this invention in an amount preferably ranging from 0.001 to 20 wt %, more preferably from 0.01 to 16 wt %, and still more preferably from 0.1 to 12 wt % of the total amount of the composition.

Drugs, the second application of the present invention, may be administered orally or topically in forms of formulation appropriate for specific applications, including liquid forms such as a liquid, injection, spray, emulsion, and like type; solid forms such as a tablet, powder, granule, capsule, spray, and like type; dermatological preparations such as ointments; and topical agents such as a suppository and like type.

Formula (1) may be present in the medicament manufactured according to the invention in an amount ranging from 0.001 to 30 wt %, preferably from 0.01 to 20 wt % and more preferably from 0.1 to 10 wt % of the total amount of the composition. The dosage is suitably varied depending on the patients' age and weight, application routes, severity of the disease, and ongoing procedure and cannot be specified, but the administered level, though not restricted, may generally be about 4 to 40 ml per day in portions 1 or 2 to 3 times per day.

The compositions used in this invention may also be formulated with Formula(1) along with other customary ingredients used in cosmetics, drugs, foods, and the like, at a level of not adversely affecting the present invention.

For example, for the cosmetics, these additives include oily substances, powders, surfactants, humectants, viscosity modifiers, lower alcohols, film-formers, UV absorbers, metal chelating agents, organic amine derivatives, pH adjusting agents, medicinally-active ingredients, polysaccharides, preservatives, vitamins, other whitening agents, antioxidants, flavoring ingredients, water, and the like.

The oily components include, for example, natural oils and fats such as jojoba oil, olive oil, avocado oil, castor oil, palm oil, beef tallow, hardened oils, and the like, and derivatives thereof; waxes such as carnauba wax, beeswax, lanolin, and the like ; hydrocarbons such as liquid paraffin, microcrystalline wax, squalane, and the like; higher fatty acid such as stearic acid and the like; higher fatty alcohols such as cetyl alcohol, stearyl alcohol, and the like; esters such as glyceryl trioctanoate, isopropyl myristate, diisostearyl malate, gryceryl di-2-heptylundecanoate, trimethylolpropane tri-2-ethylhexanoate, di-2-ethylbexyl sebacate, and the like; essential oils such as mentha oil, rose oil, citronellal, and the like; silicones such as dimethylpolysiloxane, decamethylcyclopentasiloxane, and the like .

These oily components may be incorporated in the cosmetics at levels suitably selected depending on the form, formulation type, and the like therein, usually ranging in a proportion from 0.1 to 95 % of the total amount of the cosmetic.

The typical powders used include, for example, talc, mica, kaolin, silicon dioxide, zinc oxide, titanated mica, titanium oxide, iron oxide, nylon powder, and the like .

The surfactants include, for example, nonionic surfactants such as polyoxyethylene alkyl ethers, polyoxyethylene fatty acid esters, polyoxyethylene- sorbitan fatty acid esters, glyceryl fatty acid esters, polyoxyethylene hydrogenated castor oil, polyoxyethylene sorbitol fatty acid esters, and the like; anionic surfactants such as sodium palmitate and the like; cationic surfactants such as stearyl trimethyl ammonium chloride, and the like; amphoteric surfactants such as sulfo-betaine, and the like.

The humectants include, for example, glycerine, 1,3-butyleneglycol, polyethylene glycol, and the like.

The viscosity modifiers include, for example, aqueous soluble polymers such as carboxyvinylpolymer, carboxymethylcellulose, polyvinyl alcohol, and the like; and clay minerals such as bentonite and the like.

The UV absorbers include , for example, p-aminobenzoic acid (hereafter PABA), glyceryl p-aminobenzoate, ethyl-dihydoxypropylc p-aminobenzoate, octylmethoxy cinnamate, 2-ethoxycthyl p-methoxycinnamate, 2,4-dihydroxybenzophenone, 2-hydroxy-4-methoxybenzophenone, 2-hydroxy-4-methoxy-4-metylbenzophenone, 2-hydroxy-4-methoxy-4-metylbenzophonone-5-sulfonate, ethyl urocanate, 2-phenyl-5-methylbenzoxazole, 4-methoxy-4-t-butyldibenzoylmethane, ethylhexyl p-methoxycinnamate, and the like.

The chelating agents include, for example, tetrasodium edetate, citric acid, and the like. The lower alcohols include , for example, ethanol and the like. The amines include, for example, monoethanolamine, triethanolamine, and the like. The pH adjusting agents include, for example, buffer agents such as lactic acid-sodium lactate, citric acid-sodium citrate, and the like.

The medicinally-effective ingredients include, for example, pantothenyl ethyl ether, glycyrrhizinate salts, and the like. The vitamins include, for example, vitamin C and it's derivatives, vitamin E an it's derivatives, and the like. The antioxidants include, for example, tocopherols, dibutylhydroxytoluene, propyl gallate, and the like.

The polysaccharides include, for example, erythritol, sucrose, hyaluronic acid, and the like. The preservatives include, for example, Ethylparaben (ethyl p-hydroxybenzoate) Butylparaben (n-butyl p-hydroxybenzoate), sodium benzoate, and the like. The whitening agents include, for example, ascorbic acid and it's derivatives, kojic acid and it's derivatives, glycyrrhiza extract, hydroquinone and it's derivatives, glutathione, and the like.

The drugs may further contain various additives such as excipients, stabilizers, humectants, emulsifiers, absorption inducers, pH adjusting agents, surfactants, diluents, carriers, and the like. The additives include, for example, polysaccharides such as starch and lactose; magnesium sulfate, talc, gelatin, cellulose derivatives such as hydroxypropylcellulose, vegetable oils such as soybean oil and sesame oil, animal oil, synthetic oil, rubber, aq. solutions such as physiological saline, alcohols such as ethanol, 1,3-butylene glycol, polyalkylene glycol, and the like, further including components selected from those described elsewhere that can be formulated in the cosmetics.

The foregoing compositions used in the present invention may be prepared by a conventional procedure in any desired form and formulation type by blending the essential component [Formula (1)] with one or more of the above mentioned formulation ingredients,

### Examples

The invention will be further illustrated via examples as follows; the amounts of components are given in wt% of the total amount of a given composition unless otherwise noted.

### Example 1 Melanogenesis Inhibitory Effect

### Melanogenesis Inhibitory Effect in B 16 Melanoma Cells

Melanogenesis inhibitory activity was evaluated using B 16 IOF7 melanoma cells (obtained from the Akita Research Institute of Food and Brewing, Japan), which are melanogenic cells of mouse cells, by application to the cells of an isolated form of Formula (1) described above in a solution diluted with the couture medium, thereby observing the production of melanin. The B 16 cells, cultivated to a 70% confluent level in a flask, were washed with PBS and then were treated with a trypsin solution to isolate the cells. The same volume of a serum-containing culture medium (E-MEM,10% FBS) as that of the trypsin was added to the flask, followed by collecting the cells and centrifuging (800rpm, 5min.). After this phase of the process, the number of cells was counted and adjusted to approximately 10⁴ cells/ml in the culture medium (E-MEM,10% FBS). A diluted solution of Formula (1) was prepared by placing 20 µL of sterile PBS on a cell culturing plate (a type for adherent cells) along with 20µL of a Formula (1) solution, thereby doubling dilution (a final concentration in the culture medium from 100µg/mL to 0.001.2 µg/mL). The control arbutin (a final concentration in the culture medium from 140µg/mL to 0.22µg/mL) in methanol was similarly diluted.
Then 80µL of the cell suspension was added to the diluted sample solution (20µL) on the plate, and the mixture was cultivated in a CO₂ incubator at 37° C. for 72 hours. Melanogenesis in the cells was observed under a microscope. In cases where melanogenesis was confirmed microscopically, the culture medium was removed, and the cell layer was treated with 300µL of 1N NaOH to dissolve the melanin, where the concentration of the melanin formed was determined from measurement of absorbance at 470nm. The levels of Formula (1) and arbutin used were varied to determine their minimum concentrations needed for inhibiting melanogenesis, against the value of the methanol-treated control as 100%. The results are shown in Table 1. A microscopic observation for Formula (1) also indicates that melanogenesis in B16 cells was inhibited more extensively by Formula (1) than by arbutin.

**Table 1**

| | Arbutin | Formula (1) |
|---|---|---|
| Minimum concentration for inhibition of melanogenesis | 0.88µg/mL | 0.045µg/mL |

Table 1 clearly demonstrates that Formula (1) is a better agent for inhibiting melanogenesis in B 16 melanoma cells compared than the arbutin melanogenesis inhibitor.

### Examples 2 and 3, Comparative Examples 1 and 2 Measurement of whitening effect

| Component | Content %, w/w |
|---|---|
| (Alcohol phase) | |
| Ethanol | 25.0 |
| Polyoxyethylene (25moles) hydrogenated castor oil | 2.0 |
| Antioxidant | small amount |
| Preservative | small amount |
| Perfume | small amount |
| Test compounds (see table2) | amounts given in Table2 |

| (Water phase) | |
|---|---|
| Glycerin | 5.0 |
| Sodium hexametaphosphate | small amount |
| Distilled water | balance |

Preparation The alcohol and water phases are prepared, mixed, and dissolved.

### Test Procedure

A test group made up of 20 men was exposed to sun light in summer for four hours per day from 11 a.m. to 1 p.m. for 2 days. Five days after the exposure to the sun light, the lotion described above was applied to the group on the brachial skin twice everyday, once each in the morning and in the evening for 5 weeks.

### Evaluation method

The test results after completion of the application were evaluated under the following standards:
Criterion Standards:
   More effective: Pigment deposition hardly visible
   Effective: Pigment deposition much lighter
   Somewhat effective: Pigment deposition lighter
   Ineffective: No change
Decision Criterion:
   A: 16 or more out of the group rated "Effective"' or "More effective".
   B: 11 to 15 out of the group rated "Effective" or "More effective".
   C: 6 to 10 out of the group rated "Effective" or 'More effective".
   D: 5 or fewer out of the group rated "Effective" or "More effective,"
   The evaluation results are shown in Table 2.

**Table 2**

| | Test Component | Content %, w/w | Decision |
|---|---|---|---|
| Example 2 | Formula (1) | 0.2 | B |
| Example 3 | Formula (1) | 1.0 | A |
| Comparative Example 1 | no addition | - | D |
| Comparative Example 2 | hydroquinone | 1.0 | C |

Table 2 clearly demonstrates that the lotions containing Formula (1) prevented pigment deposition and gave a whitening effect. On the other hand, neither Comparative Example 1 (no addition) nor Comparative example 2 (hydroquinone) was effective.

Examples of this invention are given below for the various formulas prepared by the usual process. All examples showed excellent melanogenesis inhibitory and whitening effects.

### Example 4 Cream

| Components | Content %, w/w |
|---|---|
| Stearic acid | 6.0 |
| Glyceryl monostearate | 2.0 |
| Polyoxyethylene (20moles) sorbitan monostearate | 2.0 |
| 13-Butylene glycol | 10.0 |
| Formula (1) | 5.0 |
| Isopropyl myristate | 12.0 |
| Squalane | 5.0 |
| Liquid petrolatum | 3.0 |
| Vitamin E | 0.05 |
| Sodium bisulfite | small amount |
| Preservative | small amount |
| Perfume | small amount |
| Distilled water | balance |

### Example 5 Emulsion

| Components | Content %, w/w |
|---|---|
| Stearyl alcohol | 2.0 |
| Squalane | 5.0 |
| Liquid lanolin | 3.0 |
| Trimethylolpropane trioctanoate | 2.0 |
| Glyceryl trioctanoate | 5.0 |
| Glyceryl monooleate | 2.0 |
| Polyoxyethylene (20 moles) sorbitan monostearate | 1.0 |
| Vitamin C | 0.1 |
| Preservative | small amount |
| Formula (1) | 8.0 |
| p-Aminobenzoic acid | 0.1 |
| Perfume | small amount |
| Sodium bisulfite | small amount |
| Glycerin | 5.0 |
| Carboxyvinylpolymer | 0.2 |
| Triethanolamine | 1.0 |
| Distilled water | balance |

### Example 6 Gel

| Components | Content %, w/w |
|---|---|
| Dipropylene glycol | 10.0 |
| Polyethylene glycol 1500 | 5.0 |
| Carboxyvinylpolymer | 1.0 |
| Ethanol | 2.0 |
| Polyoxyethylene (50 moles) oleylether | 1.0 |
| Potassium hydroxide | 0.15 |
| Formula (1) | 1.0 |
| Sodium 2-hydroxy-4-methoxybenzophenone sulfonate | 0.05 |
| Preservative | small amount |
| Trisodium edetate | small amount |
| Perfume | small amount |
| Distilled water | balance |

### Example 7 Essence

| Components | Content %, w/w |
|---|---|
| Glycerin | 5.0 |
| Dipropylene glycol | 10.0 |
| Ethanol | 10.0 |
| Carboxyvinylpolymer | 0.2 |
| Sodium hyaluronate | 0.5 |
| Potassium hydroxide | 0.1 |
| Polyoxyethylene (20 moles) sorbitan monooleate | 0.5 |
| Polyoxyethylene (20 moles) octyldodecanol | 1.0 |
| Olive oil | 0.15 |
| Placental extract | 0.1 |
| Formula (1) | 3.0 |
| Sodium bisulfite | small amount |
| Preservative | small amount |
| Trisodium edetate | small amount |
| Perfume | small amount |
| Distilled water | balance |

### Example 8 Pack

| Component | Content %,w/w |
|---|---|
| Polyvinyl acetate emulsion | 15.0 |
| Polyvinyl alcohol | 10.0 |
| Dipropylene glycol | 5.0 |
| Sorbitol | 5.0 |
| Tocopheryl acetate | 0.2 |
| Olive oil | 1.0 |
| Squalane | 3.0 |
| Polyoxyethylene (20 moles) sorbitan monooleate | 1.5 |
| Titanium dioxide | 5.0 |
| Talc | 10.0 |
| Ethanol | 7.0 |
| Formula (1) | 3.0 |
| Preservative | small amount |
| Perfume | small amount |
| Distilled water | balance |

### Example 9 Solution

| Component | Content %, w/w |
|---|---|
| 1,3-Butylene glycol | 6.0 |
| Glycerin | 5.0 |
| Sodium hyaluronate | 0.3 |
| Polyoxyethylene (20 moles) sorbitan monostearate. | 1.5 |
| Polyoxyethylene (20 mole) octyldodecanol | 0.5 |
| Ethanol | 15.0 |
| Formula (1) | 4.0 |
| Preservative | small amount |
| Perfume | small amount |
| Distilled water | balance |

### Example 10 Ointment

| Component | Content %, w/w |
|---|---|
| Polyoxyethylene (30) hydrogenated castor oil | 2.0 |
| Glyceryl monostearate | 10.0 |
| Squalane | 10.0 |
| Petrolatum | 40.0 |
| Cetanol | 6.0 |
| Formula (1) | 5.0 |
| 1,3-Butylene glycol | 10.0 |
| Distilled water | balance |
| Preservative | small amount |
| Perfume | small amount |

### Example 11 Foundation

| Component | Content %, W/W |
|---|---|
| Talc | 3.0 |
| Titanium dioxide | 5.0 |
| Red oxide of iron | 0.5 |
| Yellow oxide of iron | 1.5 |
| Black oxide of iron | 0.1 |
| Bentonite | 0.5 |
| Polyoxyethylene (20) sorbitan monostearate | 1.0 |
| Triethanolamine | 1.0 |
| Glycerin | 5.0 |
| Propylene glycol | 5.0 |
| Stearic acid | 2.0 |
| Glyceryl monostearate | 10.0 |
| Liquid petrolatum | 10.0 |
| Dimethylpolysiloxane 5.0 | |
| Polyoxyalkylene polysiloxane copolymer | 3.0 |
| Formula (1) | 3.0 |
| Distilled water | balance |
| Preservative | small amount |
| Perfume | small amount |

### Example 12 "Health drink"

| Components | Amounts (in 30ml) |
|---|---|
| Formula (1) | 0.085mg |
| Ginseng extract | 50mg |
| *Cordyceps* extract | 100mg |
| Ginger extract | 300mg |
| Honey | 150mg |
| Cyclic oligosaccharide | 300mg |
| Sweetener | small amount |
| Acidifier | small amount |
| Stabilizer | small amount |
| Flavoring Ingredient | small amount |

### Example 13 "Health drink"

| Components | Amounts (in 30ml) |
|---|---|
| Formula (1) | 0.5mg |
| Ginseng extract | 50 mg |
| Cordyceps extract | 100mg |
| Ginger extract | 300mg |
| Honey | 150mg |
| Cyclic oligosaccharide | 300mg |
| Sweetener | small amount |
| Acidifier | small amount |
| Stabilizer | small amount |
| Flavoring ingredient | small amount |

### Example 14 "Health food (tablet form)

| Components | Amounts (in 300mg) |
|---|---|
| Formula (1) | 0.025mg |
| Maltitol | 87.8mg |
| Trehalose | 41.2mg |
| Lactose | 16.5mg |
| Fatty acids esters of sucrose | 6.2mg |
| Dextrin | 148.275mg |

### Example 15 "Health food " ( tablet form)

| Components | Amounts (in 300mg) |
|---|---|
| Formula (1) | 0.5mg |
| Maltitol | 87.8mg |
| Trehalose | 41.2mg |
| Lactose | 16.5mg |
| Fatty acids esters of sucrose | 6.2mg |
| Dextrin | 147.8mg |

### POTENTIAL INDUSTRIAL UTILITY

As described above, the present invention can provide a substance effective for inhibiting melanogenesis and for whitening. It also describes the use of such substances for preventing, ameliorating, and treating skin pigmentation such as spots, freckles, and liver spots caused by sunburn and the like.

## Claims

1. Use of an ergosterol derivative represented by the following Formula (1), 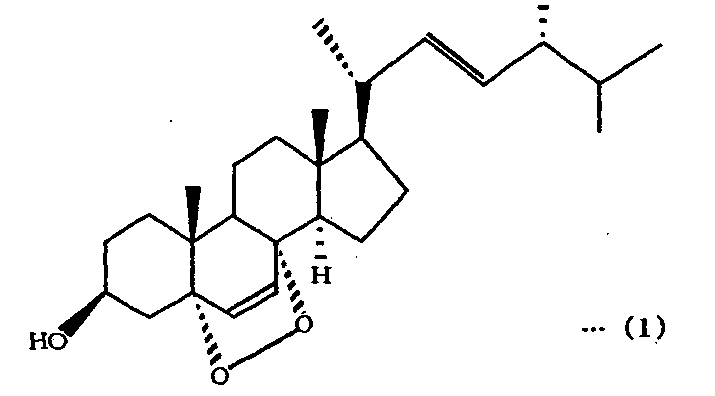 in the manufacture of a medicament for preventing, treating or ameliorating skin pigmentation.

2. A cosmetic method for whitening skin comprising applying a composition comprising an ergasterol derivative represented by formula (1) to the skin to be whitened.

## Patentansprüche

1. Verwendung eines durch die folgende Formel (1) dargestellten Ergosterolderivats 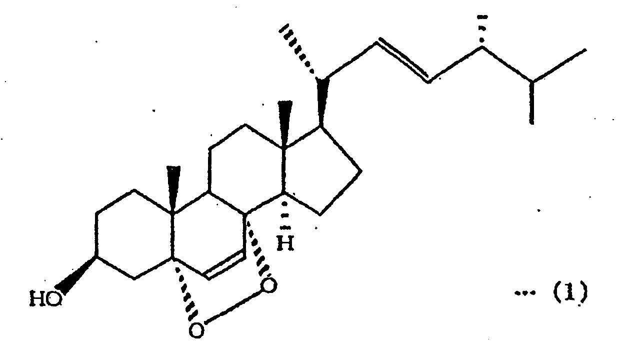 bei der Herstellung eines Medikaments für die Prävention, Behandlung oder Verbesserung von Hautpigmentierung.

2. Kosmetisches Verfahren zum Aufhellen der Haut, das Folgendes aufweist, nämlich Anwenden einer Zusammensetzung, die ein durch die Formel (1) dargestelltes Ergosterolderivat aufweist auf die aufzuhellende Haut.

## Revendications

1. Utilisation d'un dérivé d'ergostérol représenté par la formule (1) suivante 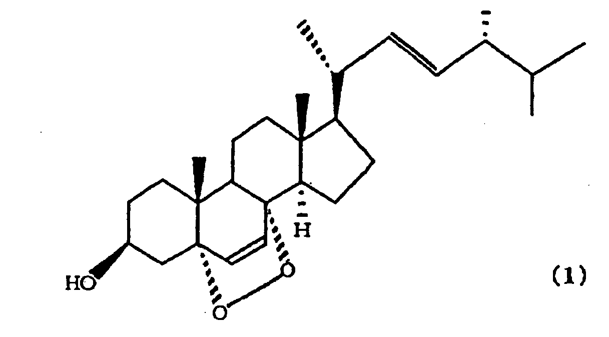 dans la fabrication d'un médicament pour la prévention, le traitement ou l'amélioration de la pigmentation de la peau.

2. Procédé cosmétique pour la dépigmentation de la peau, comprenant l'application sur la peau à dépigmenter d'une composition comprenant un dérivé d'ergostérol représenté par la formule (1)
